# EUROPÄISCHE PATENTANMELDUNG

(11) **EP 2 017 255 A1**
(43) Veröffentlichungstag der Anmeldung: **21.01.2009**
(21) Anmeldenummer: 07112756.7
(22) Anmeldetag: 19.07.2007
(51) Int. Cl.: C07C 67/04, C07C 67/54, C07C 69/54

(54) **Verfahren zur Herstellung von teritären Alkylestern der (Meth)Acrylsäure mit mindestens 4 Kohlenstoffatomen im Alkylrest**

(71) Anmelder: BASF SE, 67056 Ludwigshafen (DE)
(72) Erfinder: Lipowsky, Gunter, Dr., 69198 Schriesheim (DE); Wulff-Döring, Joachim, Dr., 67227 Frankenthal (DE); Zurowski, Peter, 76829 Landau (DE); Strasser, Jürgen, 67251 Freinsheim (DE)
(74) Vertreter: Fitzner, Uwe

(57) **Zusammenfassung**

Verfahren zur Herstellung von tertiären Alkylestern der (Meth)Acrylsäure mit mindestens 4 Kohlenstoffatomen im Alkylrest, umfassend die Verfahrenschritte:
(1) Umsetzung von (Meth)Acrylsäure mit mindestens einem Olefin der allgemeinen Formel I: 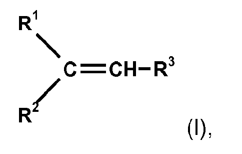 worin R¹ und R² = Alkyl-Rest und R³ = Wasserstoffatom, Alkylrest, Cycloalkyrest, Arylrest, Alkyl-Cycloalkyl-Rest, Alkyl-Aryl-Rest, Cyclolkyl-Aryl-Rest, oder Alkyl-Cycloalkyl-Aryl-Rest;
in homogener Phase in Gegenwart eines sauren Katalysators in einem Reaktor,
(2) Austragen des resultierenden Reaktionsgemischs (1) aus dem Reaktor,
(3) Auftrennen des ausgetragenen Reaktionsgemischs (2) in ein Gemisch (3.1), das den Katalysator enthält, und ein Gemisch (3.2), das den tertiären Alkylester der (Meth)Acrylsäure mit mindestens 4 Kohlenstoffatomen im Alkylrest enthält, und
(4) Abtrennen des tertiären Alkylesters der (Meth)Acrylsäure mit mindestens 4 Kohlenstoffatomen im Alkylrest aus dem Gemisch (3.2);
wobei
(5) im Verfahrenschritt (4) mindestens eine Verbindung S, die mindestens eine N-Oxyl-Gruppe enthält, als Stabilisator zugesetzt wird.

## Beschreibung

### Gebiet der Erfindung

Die vorliegende Erfindung betrifft ein neues Verfahren zur Herstellung von tertiären Alkylestern der (Meth)Acrylsäure mit mindestens 4 Kohlenstoffatomen im Alkylrest.

### Stand der Technik

Im Rahmen der vorliegenden Erfindung ist unter dem Ausdruck »(Meth)Acrylsäure« die Kurzfassung von »Acrylsäure oder Methacrylsäure« sowie »Acrylsäure und Methacrylsäure« zu verstehen. Demgemäß ist unter dem Ausdruck »(Meth)Acrylsäureester« die Kurzfassung von »Acrylsäureester oder Methacrylsäureester« sowie »Acrylsäureester und Methacrylsäureester« zu verstehen.

Ein Verfahren zur Herstellung von tertiären Alkylestern der (Meth)Acrylsäure mit 4 bis 8 Kohlenstoffatomen im Alkylrest, das die Verfahrenschritte:
(1) Umsetzung von (Meth)Acrylsäure mit mindestens einem Olefin der allgemeinen Formel: 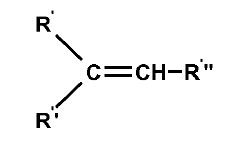 worin R' und R" gleich oder voneinander verschieden sein können und für Methyl oder Ethyl stehen und R"" für Wasserstoffatom, Methyl oder Ethyl steht; in homogener Phase in Gegenwart eines sauren Katalysators in einem Reaktor,
(2) Austragen des resultierenden Reaktionsgemischs (1) aus dem Reaktor,
(3) Auftrennen des ausgetragenen Reaktionsgemischs (2) in ein Gemisch (3.1), das den Katalysator enthält, und ein Gemisch (3.2), das den tertiären Alkylester der (Meth)Acrylsäure mit 4 bis 8 Kohlenstoffatomen im Alkylrest enthält, und
(4) Abtrennen des tertiären Alkylesters der (Meth)Acrylsäure mit 4 bis 8 Kohlenstoffatomen im Alkylrest aus dem Gemisch (3.2)
umfasst, ist aus der deutschen Patentanmeldung DE 100 36 959 A1 bekannt. Hierin wird vorgeschlagen, die Umsetzung im Verfahrenschritt (1) in der Gegenwart von Stabilisatoren wie Hydrochinon, Hydrochinonmonomethylether, p-Benzochinon, p-Nitrosophenol (PNP), Phenothiazin (PTZ), 4-Hydroxy-2,2,6,6-tetramethyl-1-oxyl-piperidin (OH-Tempo) und/oder Methylenblau durchzuführen.

Es hat sich aber gezeigt, dass OH-Tempo unter den gegebenen Reaktionsbedingungen von den sauren Katalysatoren, insbesondere insbesondere von Schwefelsäure, rasch zersetzt wird. Die Zersetzung wird noch durch die Gegenwart von PTZ beschleunigt. OH-Tempo kommt daher - wenn überhaupt - nur in sehr eingeschränktem Maße als Stabilisator bei der Umsetzung im Verfahrenschritt (1) in Betracht. Am wirksamsten erweist sich hier PTZ, insbesondere in Kombination mit Hydrochinonmonomethylether.

Bei dem bekannten Verfahren müssen bei der Aufarbeitung in dem Verfahrenschritte (3) und (4) Stabilisatoren verwendet werden. Während im Verfahrenschritt (3) das Gemisch (3.1) noch den bei der Umsetzung verwendeten Stabilisator enthält, ist das abgetrennte Gemisch (3.2) weit gehend oder völlig stabilisatorfrei und muss daher für den Verfahrenschritt (4) wieder stabilisiert werden.

Nach der DE 100 36 959 A1 werden als Stabilisatoren Gemische aus PTZ und PNP eingesetzt. PNP weist aber bekanntermaßen Nachteile auf. So ist es selbstentzündlich, giftig, explosiv und vergleichsweise teuer. Es kann jedoch hierauf nicht ohne weiteres verzichtet werden, weil die stabilisierende Wirkung von PTZ bei der Aufarbeitung zu wünschen übrig lässt, so dass die Gefahr der Polymerisation in den Destillationskolonnen und des Fouling im Sumpf besteht.

### Aufgabe

Aufgabe der vorliegenden Erfindung ist es, ein neues Verfahren zur Herstellung von tertiären Alkylestern der (Meth)Acrylsäure mit mindestens 4 Kohlenstoffatomen im Alkylrest bereitzustellen, das die Verfahrenschritte:
(1) Umsetzung von (Meth)Acrylsäure mit mindestens einem Olefin der allgemeinen Formel 1: 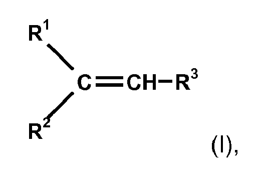 worin R¹ und R² gleich oder voneinander verschieden sein können und für einen Alkylrest stehen und R³ für ein Wasserstoffatom, einen Alkylrest, einen Cycloalkylrest, einen Arylrest, einen Alkyl-Cycloalkyl-Rest, der über ein aliphatisches oder ein cycloaliphatisches Kohlenstoffatom mit dem olefinisch ungesättigten Kohlenstoffatom verknüpft ist, einen Alkyl-Aryl-Rest, der über ein aliphatisches oder ein aromatisches Kohlenstoffatom mit dem olefinisch ungesättigten Kohlenstoffatom verknüpft ist, einen Cycloalkyl-Aryl-Rest, der über ein cycloaliphatisches oder ein aromatisches Kohlenstoffatom mit dem olefinisch ungesättigten Kohlenstoffatom verknüpft ist, oder einen Alkyl-Cycloalkyl-Aryl-Rest, der über ein aliphatisches, ein cycloaliphatisches oder ein aromatisches Kohlenstoffatom mit dem olefinisch ungesättigten Kohlenstoffatom verknüpft ist, steht;
   in homogener Phase in Gegenwart eines sauren Katalysators in einem Reaktor,
(2) Austragen des resultierenden Reaktionsgemischs (1) aus dem Reaktor,
(3) Auftrennen des ausgetragenen Reaktionsgemischs (2) in ein Gemisch (3.1), das den Katalysator enthält, und ein Gemisch (3.2), das den tertiären Alkylester der (Meth)Acrylsäure mit mindestens 4 Kohlenstoffatomen im Alkylrest enthält, und
(4) Abtrennen des tertiären Alkylesters der (Meth)Acrylsäure mit mindestens 4 Kohlenstoffatomen im Alkylrest aus dem Gemisch (3.2)
umfasst und das die Nachteile des Standes der Technik nicht mehr länger aufweist. Insbesondere soll es das neue Verfahren gestatten, ohne den Einsatz von PNP den tertiären Alkylester der (Meth)Acrylsäure von den übrigen Bestandteilen des Reaktionsgemischs abzutrennen und in besonders hoher Reinheit und in besonders hoher Ausbeute zu isolieren, ohne dass es dabei zur Polymerisation, Fouling und/oder Rückspaltung kommt.

### Erfindungsgemäße Lösung

Demgemäß wurde das neue Verfahren zur Herstellung von tertiären Alkylestern der (Meth)Acrylsäure mit mindestens 4 Kohlenstoffatomen im Alkylrest gefunden, das die Verfahrenschritte:
(1) Umsetzung von (Meth)Acrylsäure mit mindestens einem Olefin der allgemeinen Formel I: 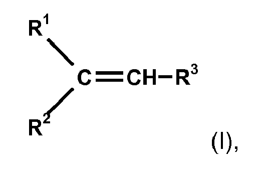 worin R¹ und R² gleich oder voneinander verschieden sein können und für einen Alkylrest stehen und R³ für ein Wasserstoffatom, einen Alkylrest, einen Cycloalkyrest, einen Arylrest, einen Alkyl-Cycloalkyl-Rest, der über ein aliphatisches oder ein cycloaliphatisches Kohlenstoffatom mit dem olefinisch ungesättigten Kohlenstoffatom verknüpft ist, einen Alkyl-Aryl-Rest, der über ein aliphatisches oder ein aromatisches Kohlenstoffatom mit dem olefinisch ungesättigten Kohlenstoffatom verknüpft ist, einen Cyclolkyl-Aryl-Rest, der über ein cycloaliphatisches oder ein aromatisches Kohlenstoffatom mit dem olefinisch ungesättigten Kohlenstoffatom verknüpft ist, oder einen Alkyl-Cycloalkyl-Aryl-Rest, der über ein aliphatisches, ein cycloaliphatisches oder ein aromatisches Kohlenstoffatom mit dem olefinisch ungesättigten Kohlenstoffatom verknüpft ist, steht;
   in homogener Phase in Gegenwart eines sauren Katalysators in einem Reaktor,
(2) Austragen des resultierenden Reaktionsgemischs (1) aus dem Reaktor,
(3) Auftrennen des ausgetragenen Reaktionsgemischs (2) in ein Gemisch (3.1), das den Katalysator enthält, und ein Gemisch (3.2), das den tertiären Alkylester der (Meth)Acrylsäure mit mindestens 4 Kohlenstoffatomen im Alkylrest enthält, und
(4) Abtrennen des tertiären Alkylesters der (Meth)Acrylsäure mit mindestens 4 Kohlenstoffatomen im Alkylrest aus dem Gemisch (3.2)
   umfasst und bei dem
(5) im Verfahrenschritt (4) mindestens eine Verbindung S, die mindestens eine N-Oxyl-Gruppe enthält, als Stabilisator zugesetzt wird.

Im Folgenden wird das neue Verfahren zur Herstellung von tertiären Alkylestern der (Meth)Acrylsäure mit mindestens 4 Kohlenstoffatomen im Alkylrest der Kürze halber als »erfindungsgemäßes Verfahren« bezeichnet.

### Vorteile der Erfindung

Im Hinblick auf den Stand der Technik war es überraschend und für den Fachmann nicht vorhersehbar, dass die Aufgabe, die der vorliegenden Erfindung zu Grunde lag, mit Hilfe des erfindungsgemäßen Verfahrens gelöst werden konnte.

Insbesondere war es überraschend, dass es das neue Verfahren gestattete, ohne den Einsatz von PNP den tertiären Alkylester der (Meth)Acrylsäure mit mindestens 4 Kohlenstoffatomen im Alkylrest von den übrigen Bestandteilen des Reaktionsgemischs abzutrennen und in besonders hoher Reinheit und in besonders hoher Ausbeute zu isolieren, ohne dass es dabei zur Polymerisation, Fouling und/oder Rückspaltung kam.

Überraschenderweise konnte auch noch ein zusätzlicher stabilisierender Effekt bei der Umsetzung im Verfahrenschritt (1) erzielt werden, wenn die Produkte, die im Verfahrenschritt (4) vom tertiären Alkylester der (Meth)Acrylsäure mit mindestens 4 Kohlenstoffatomen im Alkylrest abgetrennt worden waren, wieder in den Reaktor zurückgeführt wurden. Dieser zusätzliche stabilisierende Effekt bewirkte eine weitere Verbesserung der Ausbeute.

### Ausführliche Beschreibung der Erfindung

Das erfindungsgemäße Verfahren dient der Herstellung von tertiären Alkylestern der (Meth)Acrylsäure mit mindestens 4 Kohlenstoffatomen, insbesondere 4 bis 8 Kohlenstoffatomen, im Alkylrest. Im Folgenden werden die tertiären Alkylester der (Meth)Acrylsäure mit mindestens 4 Kohlenstoffatomen im Alkylrest der Kürze halber als »Zielester« bezeichnet.

Beispiele geeigneter Zielester sind Acrylsäure- und Methacrylsäure-tert-butylester, -2-methyl-but-2-ylester, -2-methyl-pent-2-ylester, -2-methyl-hex-2-ylester, -2-methyl-hept-2-ylester, -2-methyl-1-cyclohexyl-prop-2-ylester, -2-methyl-1-phenyl-prop-2-ylester, -2-methyl-but-2-ylester, -3-methyl-pent-3-ylester, -3-methyl-hex-3-ylester, -3-methyl-hept-3-ylester, -3-methyl-oct-3-ylester, -2-methyl-1-cyclohexyl-but-2-ylester, -2-methyl-1-phenyl-but-2-ylester, -3-methyl-pent-3-ylester, -3-ethyl-pent-3-ylester, -3-propy-pent-3-ylester, -1,1-diethylpent-1-ylester, -1,1-diethyl-hex-1-ylester, -3-(cyclohexylmethyl)-pent-3-ylester und -3-(phenylmethyl)-pent-3-ylester, insbesondere Acrylsäure-tert-butylester oder tert-Butylacrylat (TBA) und Methacrylsäure-tert-butylester oder tert-Butylmethacrylat (TBMA).

Das erfindungsgemäße Verfahren geht im Verfahrenschritt (1) von der Umsetzung von (Meth)Acrylsäure mit mindestens einem, insbesondere einem, Olefin der allgemeinen Formel I aus:

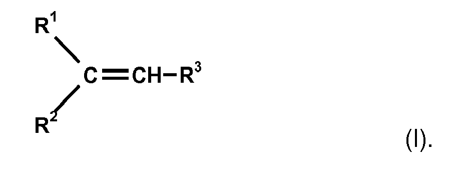

In der allgemeinen Formel I sind R¹ und R² gleich oder voneinander verschieden und stehen für einen Alkyl-Rest, vorzugsweise mit 1 bis 10, bevorzugt mit 1 bis 6 und besonders bevorzugt mit 1 bis 4 Kohlenstoffatomen, insbesondere aber für Methyl oder Ethyl.

In der allgemeinen Formel I steht R³ für
- ein Wasserstoffatom;
- einen Alkylrest vorzugsweise mit 1 bis 20, bevorzugt mit 1 bis 10 und besonders bevorzugt mit 1 bis 4 Kohlenstoffatomen, ganz besonders bevorzugt Methyl, Ethyl, Propyl, Isopropyl, n-Butyl, Isobutyl oder tert.-Butyl, insbesondere Methyl oder Ethyl;
- einen Cycloalkyrest vorzugsweise mit 4 bis 20, bevorzugt mit 5 bis 16 und besonders bevorzugt mit 6 bis 10 Kohlenstoffatomen, insbesondere Cyclohexan und einen Rest, der sich von Norbornan, Bicyclo[2.2.2]octan, Decalin, Hydrindan oder Adamantan ableitet;
- einen Arylrest vorzugsweise mit 6 bis 30, bevorzugt mit 6 bis 20 und besonders bevorzugt mit 6 bis 14 Kohlenstoffatomen, insbesondere Phenyl oder einen Rest, der sich von Naphthalin oder Phenanthren ableitet;
- einen Alkyl-Cycloalkyl-Rest, der über ein aliphatisches oder ein cycloaliphatisches Kohlenstoffatom mit dem olefinisch ungesättigten Kohlenstoffatom verknüpft ist, vorzugsweise mit 5 bis 30, bevorzugt mit 7 bis 20 und besonders bevorzugt mit 7 bis 10 Kohlenstoffatomen, insbesondere einen Rest, der sich von Methylcyclohexan, Ethylcyclohexan, Propylcyclohexan, n-Butylcyclohexan, Isobutylcyclohexan oder tert.- Butylcyclohexan ableitet;
- einen Alkyl-Aryl-Rest, der über ein aliphatisches oder ein aromatisches Kohlenstoffatom mit dem olefinisch ungesättigten Kohlenstoffatom verknüpft ist, vorzugsweise mit 7 bis 30, bevorzugt mit 7 bis 20 und besonders bevorzugt mit 7 bis 10 Kohlenstoffatomen, insbesondere einen Rest, der sich von Toluol, Xylol, Ethylbenzol, Propylbenzol, Isopropylbenzol, n-Butylbenzol, Isobutylbenzol oder tert.-Butylbenzol ableitet;
- einen Cycloalkyl-Aryl-Rest, der über ein cycloaliphatisches oder ein aromatisches Kohlenstoffatom mit dem olefinisch ungesättigten Kohlenstoffatom verknüpft ist, vorzugsweise mit 10 bis 30, bevorzugt mit 10 bis 20 und besonders bevorzugt mit 10 bis 16 Kohlenstoffatomen, insbesondere einen Rest, der sich von Cyclohexylbenzol ableitet; oder
- einen Alkyl-Cycloalkyl-Aryl-Rest, der über ein aliphatisches, ein cycloaliphatisches oder ein aromatisches Kohlenstoffatom mit dem olefinisch ungesättigten Kohlenstoffatom verknüpft ist, vorzugsweise mit 11 bis 30, bevorzugt 11 bis 20 und besonders bevorzugt 11 bis 16 Kohlenstoffatomen, insbesondere einen Rest, der sich von 1-Methyl-2-, -3- oder -4-cyclohexylbenzol ableitet;

insbesondere aber für ein Wasserstoffatom oder eine Methyl- oder Ethylgruppe steht.

Beispiele geeigneter Olefine I sind Isobuten, Trimethylethen (2-Methyl-2-buten), 2-Methyl-pent-2-en, 2-Methyl-hex-2-en, 2-Methyl-hept-2-en, 2-Methy-l-cyclohexyl-prop-1-en, 2-Methyl-1-phenyl-prop-1-en, 2-Methyl-but-1-en, 3-Methyl-pent-2-en, 3-Methyl-hex-3-en, 3-Methyl-hept-3-en, 3-Methyl-oct-3-en, 2-Methyl-1-cyclohexyl-but-1-en, 2-Methyl-1-phenyl-but-1-en, 2-Ethyl-but-1-en, 3-Ethyl-pent-2-en, 3-Ethyl-hex-3-en, 3-Ethy-hept-3-en, 3-Ethy-oct-3-en, 2-Ethyl-1-cyclohexyl-but-1-en und 2-Ethyl-1-phenyl-but-1-en, insbesondere Isobuten.

Die (Meth)Acrylsäure wird in homogener Phase, insbesondere in flüssiger homogener Phase, vorzugsweise in Abwesenheit eines Lösemittels mit dem Olefin I umgesetzt. Die Umsetzung erfolgt in der Gegenwart eines sauren Katalysators. Vorzugsweise ist der Katalysator in dem Reaktionsgemisch zumindest teilweise, bevorzugt vollständig, löslich.

Beispiele geeigneter Katalysatoren sind starke anorganische und organische Säuren, wie Mineralsäuren oder Sulfonsäuren. Beispiele gut geeigneter Mineralsäuren sind Schwefelsäure, Phosphorsäure und Polyphosphorsäure, insbesondere Schwefelsäure. Beispiele gut geeigneter Sulfonsäuren sind para-Toluol-, Benzol-, Dodecylbenzol- und Methansulfonsäure.

Die Menge des Katalysators kann breit variieren und richtet sich nach den Erfordernissen des Einzelfalls. Vorzugsweise liegt die Menge bei 1 bis 10 Gew.-%, bevorzugt 2 bis 5 Gew.-%, bezogen auf (Meth)Acrylsäure.

Das Olefin I kann gasförmig und/oder flüssig, insbesondere flüssig, eingesetzt werden.

Die eingesetzte (Meth)Acrylsäure kann Essigsäure, Propionsäure und/oder Wasser in geringen Mengen, vorzugsweise < 2 Gew.-%, insbesondere < 1 Gew.-%, bezogen auf die (Meth)Acrylsäure enthalten. Ein Beispiel für eine geeignete (Meth)Acrylsäure ist aus der deutschen Patentschrift DE 100 36 958 A1, Spalte 3, Absatz [0018], bekannt.

Die Reaktionspartner können in stöchiometrischen Mengen eingesetzt werden. Vorzugsweise wird ein Reaktionspartner im Überschuss verwendet. Vorzugsweise handelt sich dabei um die (Meth)Acrylsäure. Der Überschuss kann bis zu 50 Mol-% betragen.

Die Reaktionsbedingungen können breit variieren und richten sich nach den Erfordernissen des Einzelfalls. Vorzugsweise liegen die Reaktionstemperaturen bei 20 bis 400°C. Die Reaktionszeit beträgt vorzugsweise 1 bis 6 Stunden. Der Druck ist nicht kritisch; es kann Unterdruck, Überdruck oder vorzugsweise Umgebungsdruck oder leichter Überdruck, bevorzugt 100 bis 300 mbar (10⁴ bis 3 x 10⁴ Pa) angewandt werden.

Vorzugsweise wird die Umsetzung in der Gegenwart mindestens eines Inhibitors durchgeführt, der die Polymerisation der (Meth)Acrylsäure oder des Zielesters hemmt. Beispiele geeigneter Inhibitoren sind Hydrochinon, Hydrochinonmonomethylether, para-Benzochinon, para-Nitrosophenol (PNP), Phenothiazin (PTZ), N-Oxyl-Verbindungen wie 4-Hydroxyl-2,2,6,6-tetramethyl-1-oxyl-piperidin (OH-Tempo) und Methylenblau, bevorzugt PTZ, insbesondere PTZ und Hydrochinonmonomethylether.

Zusätzlich kann noch Sauerstoff in der Form von Luft oder Magerluft als Inhibitor verwendet werden.

Die Konzentration der Inhibitoren kann breit variieren und richtet sich nach den Erfordernissen des Einzelfalls. Vorzugsweise werden sie in einer Konzentration von 200 bis 10.000 ppm, bezogen auf das Gesamtgewicht von (Meth)Acrylsäure und Olefin I eingesetzt.

Die Umsetzung kann kontinuierlich oder diskontinuierlich in üblichen und bekannten Reaktoren durchgeführt werden. Ein Beispiel eines geeigneten Reaktors ist aus der deutschen Patentanmeldung DE 100 36 959 A1, Spalte 3, Absätze [0018] bis [0020], und Spalte 6, Absatz [0036], bis Spalte 7, Absatz [0042], in Verbindung mit der Figur, bekannt.

Die Zusammensetzung des resultierenden Reaktionsgemischs (1), das im Verfahrenschritt (2) aus dem Reaktor ausgetragen wird, kann durch die Zusammensetzung der Ausgangsprodukte, insbesondere durch das Verhältnis von (Meth)Acrylsäure und Olefin I, eingestellt und breit variiert werden. Beispiele für geeignete Zusammensetzungen von ausgetragenen Reaktionsgemischen (2) sind aus den deutschen Patentanmeldungen DE 100 36 959 A1, Spalte 7, Absatz [0043], und DE 100 36 959 A1, Spalte 4, Absatz [0022], bekannt.

Dabei können noch gegebenenfalls vorhandene Luft und Inertgase wie Butane sowie gegebenenfalls vorhandenes überschüssiges Olefin I separat vom Reaktionsgemisch
(1) aus dem Reaktor ausgetragen werden. Das Olefin I kann kondensiert und der Umsetzung wieder zugeführt werden.

Im weiteren Verlauf des erfindungsgemäßen Verfahrens wird das ausgetragene Reaktionsgemisch (2) im Verfahrenschritt (3) in ein Gemisch (3.1), das den Katalysator enthält, und ein Gemisch (3.2), das den Zielester enthält, aufgetrennt.

Die Auftrennung des Reaktionsgemischs (2) kann in der unterschiedlichsten Art und Weise erfolgen. Beispielsweise kann das Gemisch (3.1) abgetrennt werden, indem man das Reaktionsgemisch (2) mit Wasser einmal oder mehrmals wäscht und/oder den Katalysator mit einer wässrigen basischen Lösung (beispielsweise NaOH, KOH, wässrige Natrium- oder Kaliumcarbonatlösung oder -bicarbonatlösung) neutralisiert.

Insbesondere erfolgt die Auftrennung in ein Gemisch (3.1) und ein Gemisch (3.2) durch Destillation des Reaktionsgemischs (2), beispielsweise in einer Destillationseinheit, die einen Verdampfer, eine Kolonne und einen Kondensator umfasst. Man erhält in dieser Weise das Gemisch (3.2) als Kopfprodukt, das im Wesentlichen den Zielester sowie geringe Mengen an Carbonsäuren und leichtsiedende Bestandteile wie tert.-Butanol und Diisoolefin enthält. Außerdem erhält man das Gemisch (3.1) als Sumpfprodukt, das den Katalysator, die Hauptmenge an unumgesetzter (Meth)Acrylsäure und hochsiedende Bestandteile wie (Meth)Acrylat(co)polymerisate enthält. Das Sumpfprodukt (3.1) kann teilweise oder vollständig in den Reaktor zurückgeführt werden. Beispiele für ein besonders vorteilhaftes Verfahren und eine besonders vorteilhafte Vorrichtung zur Durchführung des Verfahrenschritts (3) sind aus der deutschen Patentanmeldung DE 100 36 98 A1, Spalte 4, Absatz [0023], und Spalte 5, Absatz [0029] bis Zeile 30, bekannt.

Im weiteren Verlauf des erfindungsgemäßen Verfahrens wird im Verfahrenschritt (4) der Zielester aus dem Kopfprodukt (3.2) abgetrennt. Vorzugsweise erfolgt dies durch eine zweistufige Destillation.

Dabei wird in der ersten Destillationsstufe (4.1) das Gemisch (3.2) in ein niedrig siedendes Destillat (4.1.1), das Leichtsieder wie restliches Olefin 1, insbesondere Isobuten, tert.-Butylacetat, tert.-Butanol und Diisoolefin, insbesondere Diisobuten, enthält, und ein Sumpfprodukt (4.1.2), das im Wesentlichen den Zielester und (Meth)Acrylsäure enthält, aufgetrennt. Ein Beispiel für ein geeignetes Verfahren zur Leichtsiederabtrennung (4.1) ist aus der deutschen Patentanmeldung DE 100 36 98 A1, Spalte 5, Absatz [0030], bis Spalte 6, Absatz [0036] bis Zeile 36, bekannt.

Das Sumpfprodukt (4.1.2) wird in der zweiten Destillationsstufe (4.2), d.h. in der Reindestillation, in ein Destillat (4.2.1), das im Wesentlichen aus dem Zielester besteht, und ein Sumpfprodukt (4.2.2) aufgetrennt. Hierfür kann eine übliche und bekannte Destillationseinheit, die einen Verdampfer, eine Kolonne und einen Kondensator umfasst, verwendet werden. Vorzugsweise wird das Sumpfprodukt (4.2.2), das im Wesentlichen (Meth)Acrylsäure enthält, teilweise oder vollständig in den Reaktor zurückgeführt.

Für das erfindungsgemäße Verfahren ist es wesentlich, dass im Verfahrenschritt (4) mindestens eine, insbesondere eine, Verbindung S, die mindestens eine, insbesondere eine, N-Oxyl-Gruppe enthält, als Stabilisator zugesetzt wird.

Vorzugsweise ist die N-Oxyl-Gruppe Bestandteil eines heterocyclischen, bevorzugt aliphatischen heterocyclischen, Rings. Insbesondere leitet sich der aliphatische heterocyclische Ring von einem Piperidin-Ring ab.

Vorzugsweise ist die N-Oxyl-Gruppe sterisch gehindert. Bevorzugt wird die sterische Hinderung dadurch bewirkt, dass die zum Stickstoffatom der N-Oxyl-Gruppe alpha-ständigen Kohlenstoffatome jeweils mindestens eine, insbesondere zwei Alkylgruppe(n) tragen. Insbesondere sind die Alkylgruppen Methylgruppen.

Vorzugsweise enthält die Verbindung S mindestens eine, insbesondere eine, 2,2,6,6-Tetramethyl-1-oxyl-piperidin-4-yl-Gruppe.

Bei dem mit der 4-Stellung der 2,2,6,6-Tetramethyl-1-oxyl-piperidin-4-yl-Gruppe verknüpften Rest kann es sich um
- ein Wasserstoffatom,
- ein Halogenatom, vorzugsweise ein Fluor-, Chlor- oder Bromatom,
- eine funktionelle Gruppe, vorzugsweise eine Hydroxylgruppe, eine Aminogruppe, eine Carbonsäuregruppe, eine Sulfonsäuregruppe, eine Phosphonsäuregruppe, eine Nitrilgruppe, eine Isocyanatgrupppe oder eine Nitrogruppe, bevorzugt eine Hydroxylgruppe, oder
- einen organischen Rest, vorzugsweise einen einbindigen, zweibindigen oder dreibindigen, vorzugsweise einbindigen oder zweibindigen, gegebenenfalls mindestens ein Heteroatom wie Silicium, Sauerstoff, Schwefel oder Stickstoff enthaltenden organischen Rest, bevorzugt mit 1 bis 40 und besonders bevorzugt 1 bis 30 Kohlenstoffatomen, wobei der organische Rest über eine Kohlenstoff-Kohlenstoff-Bindung, eine Äthergruppe, eine Estergruppe (-C(O)-O-Rest oder -O-C(O)-Rest), eine Aminogruppe, eine Amidgruppe (-NHC(O)-Rest oder -C(O)-NH-Rest) oder eine Urethangruppe (-NH-C(O)-O-Rest oder -O-C(O)-NH-Rest) mit der 4-Stellung verknüpft sein kann, wie beispielsweise ein Adipatrest oder ein Sebacatrest,
handeln. Insbesondere handelt es sich um eine Hydroxylgruppe. Demgemäß wird insbesondere 4-Hydroxy-2,2,6,6-tetramethyl-1-oxyl-piperidin (OH-Tempo) als Verbindung S eingesetzt.

Bei dem erfindungsgemäßen Verfahren kann die Verbindung S dem Gemisch (3.2) vor der ersten Destillationsstufe (4.1) zugesetzt werden. Vorzugsweise geschieht dies, indem man die Verbindung S in den Kondensator der Destillationseinheit für die Katalysatorabtrennung (Verfahrenschritt 3) eindosiert. Hierbei ist es von Vorteil, die Verbindung S in der Form einer verdünnten, bevorzugt dreiprozentigen, besonders bevorzugt zweiprozentigen und insbesondere einprozentigen Lösung in dem jeweiligen Zielester, der isoliert werden soll, zuzudosieren.

Bei dem erfindungsgemäßen Verfahren wird die Verbindung S in der ersten Destillationsstufe (4.1) zugesetzt. Vorzugsweise geschieht dies, indem man die Verbindung S in der Form einer verdünnten, bevorzugt dreiprozentigen, besonders bevorzugt zweiprozentigen und insbesondere einprozentigen Lösung in dem jeweiligen Zielester am Kopf der für die Leichtsiederabtrennung (4.1) verwendeten Kolonne zudosiert. Außerdem kann das aufzutrennende Gemisch (3.2) bereits die Verbindung S enthalten.

Demnach enthält das Sumpfprodukt (4.1.2) des erfindungsgemäßen Verfahrens nicht nur den Zielester und (Meth)Acrylsäure, sondern auch die Verbindung S. Die Konzentration der Verbindung S im Sumpfprodukt (4.1.2) kann breit variieren und richtet sich nach den Erfordernissen des Einzelfalls. Vorzugsweise wird so viel Verbindung S zudosiert, dass das Sumpfprodukt (4.1.2) 10 bis 10.000 ppm, bevorzugt 100 bis 5000 ppm und insbesondere 100 bis 1000 ppm der Verbindung S enthält.

Bei dem erfindungsgemäßen Verfahren wird die Verbindung S auch der zweiten Destillationsstufe (4.2) zugesetzt. Vorzugsweise geschieht dies, indem man die Verbindung S in der Form einer verdünnten, bevorzugt dreiprozentigen, besonders bevorzugt zweiprozentigen und insbesondere einprozentigen Lösung in dem jeweiligen Zielester dem unteren Teil der für die Reindestillation (4.2) verwendeten Kolonne zuführt.

Demnach enthält das Sumpfprodukt (4.2.2) des erfindungsgemäßen Verfahrens nicht nur den Zielester und (Meth)Acrylsäure, sondern auch die Verbindung S. Die Konzentration der Verbindung S im Sumpfprodukt (4.2.2) kann breit variieren und richtet sich nach den Erfordernissen des Einzelfalls. Vorzugsweise wird so viel Verbindung S zudosiert, dass das Sumpfprodukt (4.2.2) 100 bis 10.000 ppm, bevorzugt 200 bis 5000 ppm und insbesondere 400 bis 1000 ppm der Verbindung S enthält.

Das Sumpfprodukt (4.2.2) des erfindungsgemäßen Verfahrens wird vorzugsweise vollständig in den Reaktor zurückgeführt. Dort bewirkt die Verbindung S eine zusätzliche Stabilisierung, bevor sie durch den Katalysator abgebaut wird.

Zur zusätzlichen Stabilisierung kann noch Hydrochinonmonomethylether in den Rücklauf der Destillationskolonne zudosiert werden, so dass im Sumpfprodukt (4.2.2) eine Konzentration von 10 bis 500 ppm, insbesondere 20 bis 150 ppm Hydrochinonmonomethylether resultiert.

Das Destillat (4.2.1) der Reindestillation (4.2) besteht aus Zielester eines besonders hohen Reinheitsgrades. Vorzugsweise ist der Reinheitsgrad >99,5 Gew.-%, bevorzugt >99,8 Gew.-% und insbesondere >99,9 Gew.-%. Da es bei der Abtrennung der Zielester im Verfahrenschritt (4) - wenn überhaupt - nur zu einer sehr geringen Rückspaltung der Zielester und zu einer sehr geringen Polymerisation kommt, sind die Ausbeuten besonders hoch. Insbesondere wegen der sehr geringen Polymerisation in den Anlageteilen sind die Laufzeiten der Anlagen besonders lang.

### Beispiel

Die Herstellung von tert.- Butylacrylat (TBA) unter Verwendung von 4-Hydroxy-2,2,6,6-tetramethyl-1-oxyl-piperidin (OH-Tempo)

In einem üblichen und bekannten Reaktor mit Zuleitungen für Isobuten, Schwefelsäure, Phenothiazin (vierprozentig in TBA) und Acrylsäure (frische Acrylsäure eines Reinheitsgrades von 99,9%, stabilisiert mit Hydrochinonmonomethylether und Acrylsäure aus dem Sumpf der Reindestillation, enthaltend OH-Tempo), einem Austrag für das resultierende Reaktionsgemisch sowie einer Abgasleitung wurde Isobuten mit einem Überschuss Acrylsäure (50 Mol%) umgesetzt. Das resultierende Reaktionsgemisch, das TBA, Acrylsäure, PTZ, Hydrochinonmonomethylether und Katalysator enthielt, wurde ausgetragen. Der Katalysator wurde, wie in der DE 100 36 958 A1, Beispiel 1, der die Spalten 9 und 10 übergreifende Absatz [0057], beschrieben, aus dem ausgetragenen Reaktionsgemisch abgetrennt. Das resultierende, TBA enthaltende Destillat wurde in einem Wärmeaustauscher gekühlt, mit einer einprozentigen Lösung von OH-Tempo in TBA versetzt und einer Destillationseinheit zugeführt, die aus einem außen liegenden Umlaufverdampfer (Naturumlauf), einer Destillationskolonne mit 40 Dual-Flow-Böden (Zulauf auf Boden 22) und zwei seriell angeordneten Kondensatoren bestand. Am Kopf der Kolonne wurde ebenfalls eine einprozentige Lösung von OH-Tempo in TBA in einer solchen Menge zugeführt, so dass im Sumpf der Kolonne eine Gesamtkonzentration von OH-Tempo von 200 ppm resultierte. Die niedrig siedenden Bestandteile (Rest-Isobuten, Diisobuten und tert.-Butanol) wurden abgetrennt, und das im Wesentlichen aus Acrylsäure, TBA und OH-Tempo bestehende Sumpfprodukt wurde in einer weiteren Destillationseinheit einer Reindestillation unterworfen. Die Destillationseinheit bestand aus einem außen liegenden Rohrbündelverdampfer, einer Destillationskolonne mit 40 Dual-Flow-Böden (Zulauf auf Boden 18) und zwei seriell angeordneten Kondensatoren. Dem Mittelteil der Kolonne wurde ebenfalls eine einprozentige Lösung von OH-Tempo in TBA in einer solchen Menge zugeführt, dass im Sumpf der Kolonne eine Konzentration von OH-Tempo von 400 bis 700 ppm resultierte. Das Sumpfprodukt, das im Wesentlichen aus Acrylsäure bestand, wurde in den Reaktor zurückgeführt. Das abdestillierte TBA hatte einen Reinheitsgrad von 99,8%. Der Gehalt an OH-Tempo wurde mithilfe der Elektronenspinresonanz (ESR) gemessen; es konnte aber kein Signal detektiert werden. Der Gehalt lag daher unter der Nachweisgrenze von 0,3 ppm. Nach einer Laufzeit von 60 d wurde die Produktion beendet, aber nur weil keine Lagertanks mehr zur Verfügung standen. Da nach dieser Zeit kein Fouling in den Sümpfen und keine Ablagerungen von Polymerisaten auf den Kolonnenböden aufgetreten waren, hätte die kontinuierliche Produktion ohne weiteres problemlos fortgesetzt werden können.

## Patentansprüche

1. Verfahren zur Herstellung von tertiären Alkylestern der (Meth)Acrylsäure mit mindestens 4 Kohlenstoffatomen im Alkylrest, umfassend die Schritte:
(1) Umsetzung von (Meth)Acrylsäure mit mindestens einem Olefin der allgemeinen Formel I: 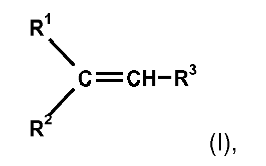 worin R¹ und R² gleich oder voneinander verschieden sein können und für einen Alkylrest stehen und R³ für ein Wasserstoffatom, einen Alkylrest, einen Cycloalkyrest, einen Arylrest, einen Alkyl-CycloalkylRest, der über ein aliphatisches oder ein cycloaliphatisches Kohlenstoffatom mit dem olefinisch ungesättigten Kohlenstoffatom verknüpft ist, einen Alkyl-Aryl-Rest, der über ein aliphatisches oder ein aromatisches Kohlenstoffatom mit dem olefinisch ungesättigten Kohlenstoffatom verknüpft ist, einen Cyclolkyl-Aryl-Rest, der über ein cycloaliphatisches oder ein aromatisches Kohlenstoffatom mit dem olefinisch ungesättigten Kohlenstoffatom verknüpft ist, oder einen Alkyl-Cycloalkyl-Aryl-Rest, der über ein aliphatisches, ein cycloaliphatisches oder ein aromatisches Kohlenstoffatom mit dem olefinisch ungesättigten Kohlenstoffatom verknüpft ist, steht;
in homogener Phase in Gegenwart eines sauren Katalysators in einem Reaktor,
(2) Austragen des resultierenden Reaktionsgemischs (1) aus dem Reaktor,
(3) Auftrennen des ausgetragenen Reaktionsgemischs (2) in ein Gemisch (3.1), das den Katalysator enthält, und ein Gemisch (3.2), das den tertiären Alkylester der (Meth)Acrylsäure mit mindestens 4 Kohlenstoffatomen im Alkylrest enthält, und
(4) Abtrennen des tertiären Alkylesters der (Meth)Acrylsäure mit mindestens 4 Kohlenstoffatomen im Alkylrest aus dem Gemisch (3.2),
**dadurch gekennzeichnet, dass**
(5) im Verfahrenschritt (4) mindestens eine Verbindung S, die mindestens eine N-Oxyl-Gruppe enthält, als Stabilisator zugesetzt wird.

2. Verfahren nach Anspruch 1, **dadurch gekennzeichnet, dass** das Stickstoffatom der N-Oxyl-Gruppe Bestandteil eines heterocyclischen Rings ist.

3. Verfahren nach Anspruch 2, **dadurch gekennzeichnet, dass** der heterocyclische Ring aliphatisch ist.

4. Verfahren nach Anspruch 3, **dadurch gekennzeichnet, dass** sich der heterocyclische aliphatische Ring von einem Piperidin-Ring ableitet.

5. Verfahren nach einem der Ansprüche 1 bis 4, **dadurch gekennzeichnet, dass** die N-Oxyl-Gruppe sterisch gehindert ist.

6. Verfahren nach Anspruch 5, **dadurch gekennzeichnet, dass** die zum Stickstoffatom der N-Oxyl-Gruppe alpha-ständigen Kohlenstoffatome jeweils mindestens eine Alkylgruppe tragen.

7. Verfahren nach Anspruch 6, **dadurch gekennzeichnet, dass** die zum Stickstoffatom der N-Oxyl-Gruppe alpha-ständigen Kohlenstoffatome jeweils 2 Alkylgruppen tragen.

8. Verfahren nach Anspruch 6 oder 7, **dadurch gekennzeichnet, dass** die Alkylgruppen Methylgruppen sind.

9. Verfahren nach Anspruch 8, **dadurch gekennzeichnet, dass** die Verbindung S mindestens eine 2,2,6,6-Tetramethyl-1-oxyl-piperidin-4-yl-Gruppe enthält.

10. Verfahren nach Anspruch 9, **dadurch gekennzeichnet, dass** die Verbindung S 4-Hydroxy-2,2,6,6-tetramethyl-1-oxyl-piperidin ist.

11. Verfahren nach einem der Ansprüche 1 bis 10, **dadurch gekennzeichnet, dass** das Gemisch (3.1) teilweise oder vollständig in den Reaktor zurückgeführt wird.

12. Verfahren nach einem der Ansprüche 1 bis 11, **dadurch gekennzeichnet, dass** das Gemisch (3.2) als Destillat abgetrennt wird.

13. Verfahren nach einem der Ansprüche 1 bis 12, **dadurch gekennzeichnet, dass** im Verfahrenschritt (4) der tertiäre Alkylester der (Meth)Acrylsäure mit mindestens 4 Kohlenstoffatomen im Alkylrest durch zweistufige Destillation aus dem Gemisch (3.2) abgetrennt wird.

14. Verfahren nach Anspruch 13, **dadurch gekennzeichnet, dass** die Verbindung S in der ersten Destillationsstufe (4.1) und der zweiten Destillationsstufe (4.2) zugesetzt wird.

15. Verfahren nach Anspruch 14, **dadurch gekennzeichnet, dass** die Verbindung S dem Gemisch (3.2) vor der ersten Destillationsstufe (4.1) zugesetzt wird.

16. Verfahren nach Anspruch 14 oder 15, **dadurch gekennzeichnet, dass** in der ersten Destillationsstufe (4.1) das Gemisch (3.2) in ein niedrig siedendes Destillat (4.1.1) und ein Sumpfprodukt (4.1.2), enthaltend den tertiären Alkylester der (Meth)Acrylsäure mit mindestens 4 Kohlenstoffatomen im Alkylrest und die Verbindung S, aufgetrennt wird.

17. Verfahren nach Anspruch 16, **dadurch gekennzeichnet, dass** das Sumpfprodukt (4.1.2) in der zweiten Destillationsstufe (4.2) in ein Destillat (4.2.1), das im wesentlichen aus dem tertiären Alkylester der (Meth)Acrylsäure mit mindestens 4 Kohlenstoffatomen im Alkylrest besteht, und ein Sumpfprodukt (4.2.2) aufgetrennt wird, das die Verbindung S enthält, aufgetrennt wird.

18. Verfahren nach Anspruch 17, **dadurch gekennzeichnet, dass** das Sumpfprodukt (4.2.2) teilweise oder vollständig in den Reaktor zurückgeführt wird.

19. Verfahren nach einem der Ansprüche 1 bis 18, **dadurch gekennzeichnet, dass** das Olefin der allgemeinen Formel I Isobuten ist.
